# EUROPEAN PATENT SPECIFICATION

(11) **EP 1 517 134 B1**
(45) Date of publication and mention of the grant of the patent: **20.04.2016**
(21) Application number: 04020880.3
(22) Date of filing: 02.09.2004
(51) Int. Cl.: G01N 21/64, G01N 33/50, G02B 21/00

(54) **Drug screening device using a Nipkow confocal scanner**
Drogenscreeningvorrichtung mit einem Nipkow confocal Scanner
Dispositif de criblage de médicament à l'aide d'un module de balayage confocal de Nipkow

(30) Priority: 22.09.2003 JP 2003329735; 22.09.2003 JP 2003329736; 01.10.2003 JP 2003342813
(43) Date of publication of application: 23.03.2005
(73) Proprietor: Yokogawa Electric Corporation, Tokyo 180-8750 (JP)
(72) Inventor: Uchida, Isao, Tokyo 180-8750 (JP); Mikuriya, Kenta, Tokyo 180-8750 (JP); Suzuki, Toshiyuki, Tokyo 180-8750 (JP); Iino, Toshio, Tokyo 180-8750 (JP); Akiyama, Takashi, Tokyo 180-8750 (JP)
(74) Representative: Henkel, Breuer & Partner

(56) References cited:
- EP-A- 1 180 679
- WO-A-00/17643
- US-A- 5 795 755
- US-A- 5 843 436
- US-A- 5 876 946
- US-A1- 2003 030 896
- US-B1- 6 179 421
- TADAKUMA H ET AL: "Imaging of single fluorescent molecules using video-rate confocal microscopy." BIOCHEMICAL AND BIOPHYSICAL RESEARCH COMMUNICATIONS. 21 SEP 2001, vol. 287, no. 2, 21 September 2001 (2001-09-21), pages 323-327, XP002312263 ISSN: 0006-291X
- FUJITA K ET AL: "Confocal multipoint multiphoton excitation microscope with microlens and pinhole arrays" OPTICS COMMUNICATIONS, NORTH-HOLLAND PUBLISHING CO. AMSTERDAM, NL, vol. 174, no. 1-4, January 2000 (2000-01), pages 7-12, XP004186831 ISSN: 0030-4018
- JAYARAMAN S ET AL: "Airway surface liquid osmolality measured using fluorophore-encapsulated liposomes." THE JOURNAL OF GENERAL PHYSIOLOGY. MAY 2001, vol. 117, no. 5, May 2001 (2001-05), pages 423-430, XP009012907 ISSN: 0022-1295

## Description

### BACKGROUND OF THE INVENTION

### 1. Field of the invention

The present invention relates to a screening device used in the discovery of a drug, or drug discovery for developing a new kind of medical product, and so on.

### 2. Description of the prior art

For example, a device for screening a cell processed by a fluorescent reagent, and so on, and described in International Patent Publication No. 2002-525603 (International Laid-Open No. WO00/17643) is well known as a screening device of this kind.

Fig. 1 is a constructional view of the screening device described in patent literature 1. This device uses a standard objective lens having a magnification power of 1 to 100 in comparison with a camera lens, and an inverted type fluorescent microscope 1 such as a Zeiss Axiovert inverted type fluorescent microscope using a light source (e.g. a 100W mercury-arc lamp or 75W xenon lamp) with power source 2. XY stage 3 for moving plate 4 in the XY directions is arranged on the microscope objective lens.

Plate 4 is a standard plate of 96 wells. A cell is injected into each well, and a reagent is added. After plate 4 is placed on microscope assembly 1, plate 4 is moved in the XY directions by XY stage 3. A fluorescent image emitted from the cell of each well is received by camera 7. An output signal of the camera is inputted to central processing unit 10, and is processed and displayed in a display format corresponding to the fluorescent image on display 12 of PC 11. Further, the record of a hard copy can be printed in printer 13.

Thus, the operating effect of the reagent with respect to the cell can be detected.

A Z-axis focal point driving mechanism 5 is a Z-axis direction moving mechanism for adjusting the focal point of the objective lens. Joystick 6 is used to manually move the stage in the XYZ directions. In addition, power source 8 for the camera, and automatic controller 9 are arranged.

However, such screening devices have the following problems.
(1) In an optical fluorescent microscope, focused images cannot be simultaneously obtained in all portions, from the upper portion to the lower portion of the cell, and a defocused dim image is obtained. Accordingly, a detailed and accurate image having a good SN ratio and able to grasp the form and degree of activity (ADMETOX: absorption, distribution, metabolism, excretion and toxicity) and so on, over the entire cell cannot be obtained.
(2) Thermal energy of the light source is strong and has a negative influence on the cell.
(3) It is necessary to adjust the focal position of the objective lens prior to observation.

EP 1 180 679 A discloses a screening device comprising a sensor section including a light source, a Nipkow system confocal scanner for converging light from said light source onto a sample of a plate and a camera, an image processing section and a central processing unit, a XYZ-drive controllable by a control mechanical section for arranging a substrate stage, which is configured to place said plate thereon, in a predetermined position with respect to said sensor section so as to observe each sample provided on said plate. Said Nipkow system confocal scanner includes a pinhole disk provided so as to pass the individual light beams through individual pinholes to the sample.

### SUMMARY OF THE INVENTION

An object of the present invention is to solve such problems and provide a screening device, as defined by the appended claims, able to obtain the entire image of the cell as a detailed and accurate (with a good SN ratio) image at high speed by observing the cell image while a Nipkow system confocal scanner is used and the focal position of the objective lens is changed in the optical axis direction.

Another object of the present invention is to provide a screening device, as defined by the appended claims, which, by reducing the amount of irradiating light of the excitation light, does not produce a negative influence by the excitation light on the cell.

Another object of the present invention is to provide a new drug screening device, as defined by the appended claims, able to observe the entire image of the cell with high resolution and a high degree of accuracy by observing the cell image while a Nipkow system confocal scanner is used and the focal position of the objective lens is changed in the optical axis direction, and able to judge the degree of activity of the cell from the obtained image, and display a judging result in an easily recognizable display format.

Another object of the present invention is to provide a new drug screening device, as defined by the appended claims, able to automatically inject the cell and the reagent into the well in accordance with a condition set in advance.

Another object of the present invention is to provide a new drug screening device, as defined by the appended claims, for calculating an optical flow from a time differential image by an arithmetic operation, and able to determine the degree of activity of the cell by making an inference for distinguishing circulatory activity within the cell and a Brownian movement on the basis of this calculation.

Further, still another object of the present invention is to provide a new drug screening device, as defined in the appended claims, able to simply mark a target well so as to easily find the optimum well, the worst well, and so on, from the plate after measurement.

Further, another object of the present invention is to provide a new drug screening device, as defined in the appended claims, having no negative influence by the excitation light on the cell by reducing the irradiating light amount.

Further, another object of the present invention is to provide a new drug screening device, as defined by the appended claims, able to grasp the effect of the reagent with respect to the cell by calculating the degree of activity (ADMETOX) of the cell on the basis of the fluorescent image of the cell, and judging whether this degree of activity is good or not.

Further, another object of the present invention is to provide a new drug screening device, as defined by the appended claims, for calculating the optical flow from the time differential image of the fluorescent image by an arithmetic operation, and determining the degree of activity of the cell by making the inference for distinguishing the circulatory activity within the cell and the Brownian movement on the basis of this calculation, and judging whether this degree of activity is good or not, and obtaining this judging result in the early stage of screening.

### BRIEF DESCRIPTION OF THE DRAWINGS

Fig. 1 is a constructional view showing one example of a conventional screening device
Fig. 2 is a constructional view showing one preferred embodiment of a screening device in accordance with the present invention.
Fig. 3 is a view showing one preferred embodiment of a confocal scanner.
Fig. 4 is a constructional view showing another preferred embodiment of the screening device in accordance with the present invention.
Fig. 5 is a schematic view showing the characteristics of the degree of activity.
Fig. 6 is an explanatory view of the plate and the well.
Fig. 7 shows a display example of a judging result of the degree of activity.
Fig. 8 shows another display example of a judging result of the degree of activity.

### DETAILED DESCRIPTION OF THE PREFERRED EMBODIMENTS

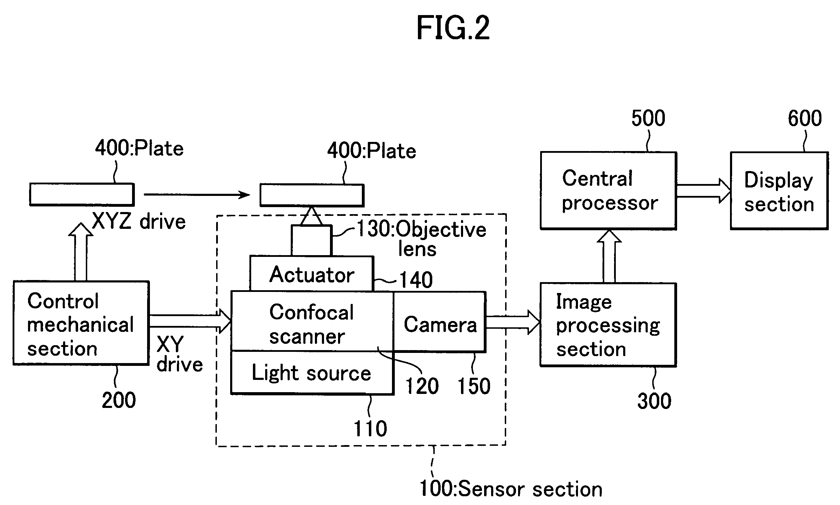

Next, the present invention, defined by the appended claims, will be explained in detail using the drawings. Fig. 2 is a constructional view showing one preferred embodiment of a screening device in accordance with the present invention.

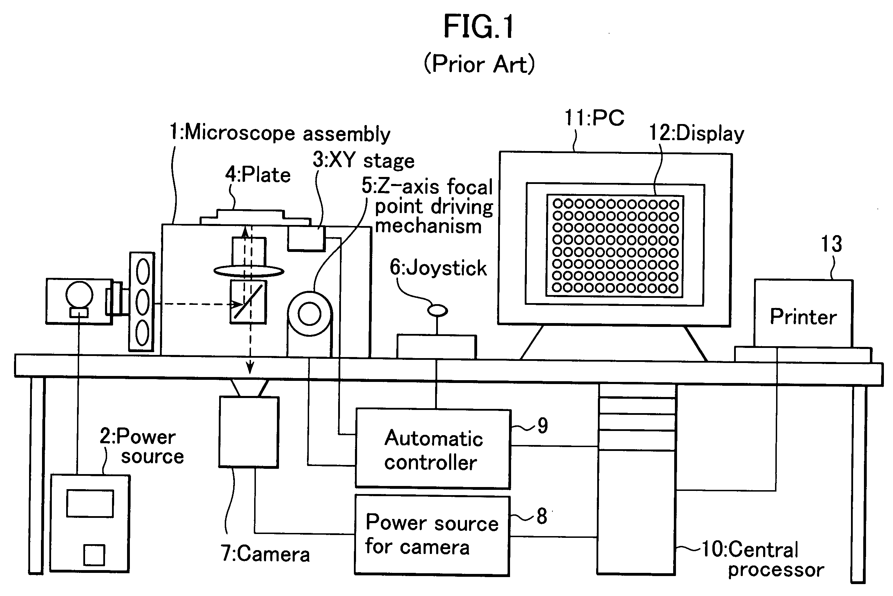

In Fig. 2, reference numerals 100, 200, and 300, designate a sensor section, a control mechanical section, and an image processing section respectively. Reference numerals 400, 500, and 600, designate a plate (corresponding to plate 4 of Fig. 1), a central processing unit, and a display section respectively.

Sensor section 100 is constructed from laser light source 110, Nipkow system confocal scanner 120, objective lens 130, focal position variable means 140 and camera 150.

A laser beam as excitation light generated from laser light source 110 is converged onto a sample of plate 400 by objective lens 130 through Nipkow system confocal scanner 120. Fluorescent light from the sample excited by the laser beam is returned to confocal scanner 120 via objective lens 130, and is inputted to camera 150. In camera 150, a fluorescent image of the XY plane of sample 401 is obtained. In this case, since a Nipkow system confocal scanner is used, a detailed and accurate (with a good SN ratio) image is obtained at high speed.

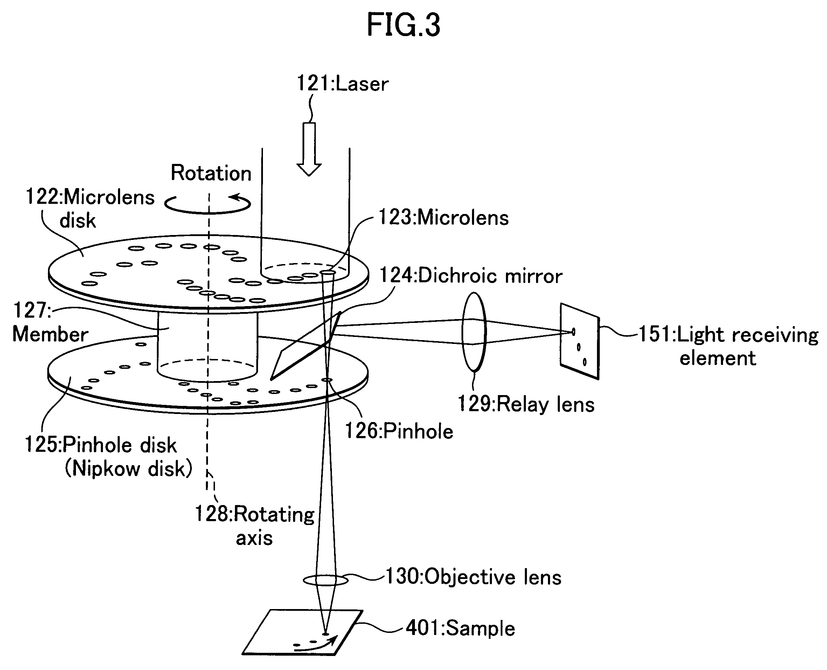

For example, Nipkow system confocal scanner 120 is constructed as shown in Fig. 3. Fig. 3 shows this construction in reverse relation to Fig. 2 with respect to the upper and lower portions. Further, objective lens 130 and light receiving element 151 of camera 150 are shown together.

In Fig. 3, laser beam 121 as the excitation light is converged to an individual light beam by each microlens 123 arranged in microlens disk 122. After the laser beam is transmitted through dichroic mirror 124, the laser beam passes through individual pinhole 126 formed in pinhole disk (also called a Nipkow disk) 125 and is converged to sample 401 by objective lens 130.

The fluorescent light generated from sample 401 again passes through objective lens 130, and is converged onto the individual pinhole of pinhole disk 125. The fluorescent light passing through individual pinhole 126 is reflected on dichroic mirror 124 , and a fluorescent image is formed on light receiving element 151 of the camera by relay lens 129.

Dichroic mirror 124 used here is designed so as to transmit excitation light 121 and reflect the fluorescent light from sample 401.

Microlens disk 122 and pinhole disk 125 are mechanically connected by member 127, and are integrally rotated around rotating shaft 128. Individual microlens 123 and pinhole 126 are arranged such that the excitation light from individual pinhole 126 formed on pinhole disk 125 scans an observing plane of sample 401. Further, an arranging plane of pinhole 126, the observing plane of sample 401 and light receiving element 151 of the camera are mutually arranged in an optically conjugate relation. Therefore, an optical sectional image of sample 401, i.e. a confocal image is formed on light receiving element 151.

In Fig. 2, focal position variable means 140 operates objective lens 130 or on the entire sensor section 100, and continuously or discontinuously moves (hereinafter also termed scans) the focal position of objective lens 130 in the Z-axis direction (optical axis direction) . For example, a Piezo actuator (simply called actuator) is used as focal position variable means 140. Hereinafter, an explanation will be given with the actuator as an example.

Control mechanical section 200 performs an XYZ direction operation for arranging a substrate stage, not shown in the figure, placing plate 400 thereon in a predetermined position with respect to sensor section 100 and moving the substrate stage suitably in the XYZ directions so as to sequentially observe each well. Control mechanical section 200 also performs an XY direction operation for adjusting the XY direction positions of the sensor section.

Image processing section 300 receives an image signal from camera 150, and performs suitable image processing and data processing for showing the degree of activity of a cell, and so on. For example, the processing also includes processing for making a statistical analysis of the fluorescent strength of the cell, kinetics, a histogram, a correlation diagram, and so on.

Differing from the conventional device, a detailed and accurate image having a good SN ratio is obtained at high speed in the present device. Accordingly, the dynamic mode of each cell, and so on, can be easily and accurately obtained.

The processed image is displayed in display section 600 by central processing unit 500.

Central processing unit 500 also appropriately controls the operations of control mechanical section 200, actuator 140 and image processing section 300.

Next, the operation of the device in such a construction will be explained. A sample, i.e. a living cell and a fluorescent marked reagent, are respectively injected into each well of plate 400 in advance. Plate 400 is moved by control mechanical section 200 and is arranged in a predetermined position on sensor section 100.

The sample is irradiated by the laser beam (excitation light) from sensor section 100, and fluorescent light is generated from the cell. This fluorescent light is formed as an image of confocal scanner 120 through objective lens 130 and actuator 140. This image (cell image) is read by camera 150.

At this time, objective lens 130 is continuously or discontinuously scanned by actuator 140 in the Z-axis direction.

In accordance with the Nipkow system confocal scanner, a slice image of the cell is obtained at high speed, and the slice image of the cell in each different sectional position can be obtained from above to below in the Z-axis direction by camera 150.

In image processing section 300, predetermined image processing is performed on the basis of plural slice images obtained by camera 150, and images of the degree of activity, and so on, over the entire cell are obtained.

In the image processing, processing for forming one two-dimensional image (overlapping image) from the plural slice images obtained by camera 150 by, for example, three-dimensional data processing, and classifying the image by color in accordance with the difference in the expression amount of fluorescent protein, and so on, are also performed. Further, Hough transform processing can be also included in the image processing.

The image processed by image processing section 300 is stored to a memory means, not shown in the figure, as necessary, and is displayed in display section 600 by central processing unit 500.

Thus , a detailed and accurate image having a good SN ratio , and not obtained in devices using the conventional fluorescent microscope, can be observed.
It is to be understood that the present invention is not restricted to the foregoing embodiments, rather, many other alterations and modifications thereof may be made without departing from the scope of the appended claims.

For example, in the preferred embodiment, three-dimensional objective lens 130 is scanned in the Z-axis direction by Piezo actuator 140, but three-dimensional processing may be also performed using multiheads with far, mid- and close focal positions, and so on.

The focal position of the objective lens with respect to the sample may be also moved by voltage control by inserting a varifocal lens of the voltage control between objective lens 130 and confocal scanner 120 without operating objective lens 130 with the Piezo actuator.

Further, according to the invention, excitation is performed by a laser beam. In an example which does not fall within the scope of the present invention, fluorescent light may also be generated from the sample by utilizing 2-photon absorption and irradiating infrared light as the excitation light and causing 2-photon absorption. In accordance with this construction, it is sufficient to irradiate the infrared light having reduced light energy to the cell so that the influence of light on the cell can be almost eliminated.

Further, the image of the sample may be also picked up by the camera by dyeing the cell using quantum dots , performing excitation with a short wavelength laser beam, and giving the filter of the camera spectroscopic characteristics with a discriminating function. Thus, sensitivity and discriminating ability can be easily improved.

Further, in an example which does not fall within the scope of the present invention, the Nipkow system confocal scanner's output image may also be detected by using a line sensor. In accordance with this construction, a coincident property and a higher resolution property can be simultaneously achieved. In this case, a multicolor line sensor may be also used as the line sensor.

An image (of a long focal depth corresponding to a scanning thickness which is called a long focal image) obtained by exposing sectional image information of the sample (cell) obtained by scanning the objective lens in the optical axis direction by a required depth within the same frame period of the camera may also be used as the image utilized in image processing section 300.

In accordance with the present invention explained above, there are the following effects.
(1) The Nipkow system confocal scanner is used, the focal position of the objective lens is changed in the optical axis direction and the image of the sample is observed. Therefore, a detailed and accurate (with a good SN ratio) image can be obtained at high speed.
(2) In comparison with the conventional case, the thermal energy of the excitation light is weak and the image can be observed without having a negative influence on the cell.
(3) It is not necessary to adjust the focal position of the objective lens prior to the observation as in the conventional case.

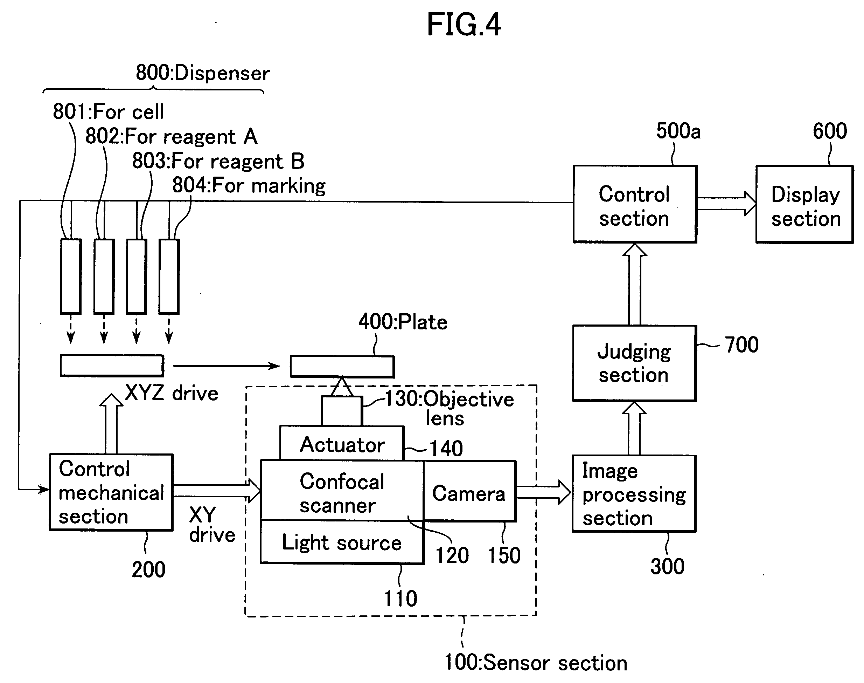

Fig. 4 is a constructional view of another preferred embodiment in the present invention. This new drug screening device realizes the following points.
(1) The Nipkow system confocal scanner is used and the image of the cell is observed while the focal position of the objective lens is changed in the optical axis direction. Thus, the entire image of the cell is observed with high resolution and a high degree of accuracy. The degree of activity of the cell is judged from the obtained image, and the judging result is displayed in an easily recognized display format.
(2) The cell and the reagent can be automatically injected into the well in accordance with a condition set in advance.
(3) An optical flow is calculated from a time differential image by an arithmetic operation, and the degree of activity of the cell can be determined by making an inference for distinguishing circulatory activity within the cell and a Brownian movement on the basis of this calculation.
(4) A target well can be simply marked to easily find out the optimum well, the worst well, and so on, from the plate after measurement.
(5) The irradiating light amount is reduced and there is no negative influence from the excitation light on the cell.

Next, Fig. 4 will be explained. In this figure, the constructions of reference numerals 100 to 400 are equal to those of Fig. 2, and their explanations are therefore omitted here. An image processed by image processing section 300 is displayed in display section 600 by control section 500a.

For example, a microcomputer is used in control section 500a which appropriately controls the operations of control mechanical section 200, actuator 140, image processing section 300, judging section 700 and dispenser 800.

Dispenser 800 has a mechanism for respectively injecting the cell (a living cell fluorescent marked in advance) and the reagent into each well of plate 400 by using, for example, an electromagnetic pump, not shown in the figure, and so on, and performing the marking on the plate after the measurement. The reagent is not limited to one kind, but various kinds of reagents such as plural reagents, reagents of the same kind but of different densities, and so on, are applied appropriately. Dispenser 801 is used for the cell injection, and dispenser 802 is used to inject reagent A. Dispenser 803 is used to inject reagent B, and dispenser 804 is used for coating with colored paint for marking. The number of dispensers for the reagents and the paint are not limited to the embodiment.

The cell and the reagent to be injected into each cell are set and stored in control section 500a in advance. The electromagnetic pumps of dispensers 801, 802, 803 are operated in accordance with this setting, and the cell and the reagent are automatically injected into each well of plate 400. At this time, plate 400 is automatically moved to a position corresponding to the injecting dispenser by control mechanical section 200 controlled by control section 500a.

Judging section 700 has a function for determining the degree of activity of the cell by using data after image processing obtained by image processing section 300. One example of the function for determining the degree of activity will next be described.

A moving vector of fine particles is extracted from the differential image between the image of time (t) and the image of time (t+Δt) after a certain time has passed. Every Δt in the whole flow of this vector is called an optical flow. In the case of plasma streaming, this optical flow is set to one direction. In the case of the Brownian movement, the flow direction is random and no movement is made at a long distance even when the movement is integrated.

Judging section 700 has an inferential portion (AI inferential engine) assembling a program for distinguishing the circulatory activity within the cell and the Brownian movement on the basis of the optical flow calculated from the time differential image. Thus, the degree of activity of the cell can be judged without error. In particular, the speed of the plasma streaming has a good relation with the degree of activity and a rapid judgment can be made in the early stage of screening.

Further, judging section 700 can also perform an output operation in a display format easily discriminated in the display section by adding a mark classified by color and corresponding to an adopted or rejected selection or the degree of activity in each well in accordance with the degree of activity, and so on.

Next, the operation of the device in such a construction will be explained. The cell and the reagent are injected into each cell of plate 400 from the dispenser in accordance with information set to control section 500a in advance. In this case, dispenser 800 and plate 400 are operated by control mechanical section 200 in accordance with the instructions of control section 500a, and are moved relatively.

After the injection into the well, plate 400 is arranged in a predetermined position on sensor section 100 by operating control mechanical section 200.

The sample of the well is irradiated by a laser beam (excitation light) from sensor section 100, and fluorescent light is generated from the cell. This fluorescent light passes through objective lens 130 and actuator 140 and is formed as an image on Nipkow system confocal scanner 120. This image (cell image) is then read by camera 150.

At this time, objective lens 130 is continuously or discontinuously scanned by actuator 140 in the Z-axis direction. Thus, a slice image of the cell is obtained by camera 150 in each different sectional position from above to below in the Z-axis direction. Each slice image obtained by the confocal scanner is a detailed and accurate image having a good SN ratio. In the present invention, since a Nipkow system confocal scanner is used as the confocal scanner, a detailed and accurate image having a good SN ratio can be obtained at higher speed.

Thus, the image is similarly measured with respect to each well. This image measurement is made at a predetermined time interval.

Image processing section 300 obtains an image of a super depth, in which the image in the depth direction of the cell is clearly and accurately displayed using plural slice images obtained by camera 150. This image is a fluorescent image in which a moving mode of the entire cell is more accurately grasped in comparison with a defocused image using the conventional fluorescent microscope irrespective of the dispersion of each cell in the Z-axis direction within the well. These image data can be appropriately stored to a memory means, which is not shown in the figure.

Hough transform may also be used in the image processing. Further, a super depth fluorescent image may be photographed by camera 150 by continuously scanning actuator 140 in the Z-axis direction.

The image data processed by image processing section 300 is transmitted to judging section 700. Judging section 700 calculates the optical flow by the AI inferential engine from the differential image between the image of time (t) and the image of time (t+Δt) after a certain time has passed. Judging section 700 then judges the degree of activity of the cell. Judging section 700 further outputs data of the display format, easily discriminated by adding a mark classified by color according to the degree of activity of each well, and so on. Further, judging section 700 also outputs information as to which well is the optimum well and the worst well.

The judging result in judging section 700 is displayed in display section 600. The judging result can be stored to a memory means, not shown in the figure, as necessary.

Control section 500a can display information such as the injecting condition of the well, for example, the kind and the density of the cell, and the reagent injected into each well, and so on, in display section 600 together with the above judging result.

Thus , a detailed and accurate image having a good SN ratio and not obtained in the device using the conventional fluorescent microscope is obtained and the degree of activity of the cell is automatically judged for each well and its judging result can be displayed in an easily recognizable display format.

On the other hand, the number of wells arranged in one plate tends to increase from 96 to 384, 1536, 6144 and 8000.

It is not easy to find the optimum well and the worst well from the plate after the measurement. There are also cases in which the optimum well and the worst well are easily found in error. Therefore, in control section 500a, only a target well of plate 400 after the measurement is marked by operating dispenser 804 for marking and control mechanical section 200 on the basis of the judging result of judging section 700. For example, colored paint is attached to the vicinity of the target well of the plate surface by dispenser 804.

In accordance with the new drug screening device as shown in Fig. 4, the following effects are shown.
(1) Since the confocal scanner is used and the image of the sample is observed by changing the focal position of the objective lens in the optical axis direction, any cell can be entirely observed with high resolution and high accuracy.
(2) In comparison with the conventional case, the thermal energy of the excitation light is weak and the cell can be observed without having a bad influence on the cell.
(3) Since the degree of activity of the cell can be automatically judged and its result is classified by color and is displayed, and so on, the state of the cell and the drug effects can be grasped easily.
(4) Since the target well such as the optimum well, the worst well, and so on, on the plate can be marked, the target well can be easily found on the plate after the observation.
(5) The trend of the dynamic mode of the cell can be easily grasped.
(6) The injecting condition of the cell and the reagent into the well is variously set, and the cell and the reagent can be easily automatically injected accordingly.

The next preferred embodiment shows a new drug screening device for solving the following problems.
(1) A disadvantage of the conventional device is that no difference between the plasma streaming of living cells and the Brownian movement of an organelle within dead cells can be distinguished, and it is impossible to judge whether it is a living or a dead cell in the image processing. Therefore, in the actual method, feeding habits are judged in a state for distinguishing whether the cell is dead or living after sufficient time has passed. Therefore, a problem exists in that it takes time to observe the effect of the reagent.
(2) The effect of the reagent with respect to the cell is judged visually by an operator. Therefore, problems exist in that the judgment is complicated and there are errors in the judgment, and so on.
(3) In the optical fluorescent microscope, no focused image is obtained in all portions of the cell from above to below, and the cell can be inspected only by a defocused dim image. Accordingly, a problem exists in that the shape mode and the dynamic mode over the entire cell cannot be grasped well.

The preferred embodiment of this invention has the following points as objects to solve such problems. (1) The effect of the reagent with respect to the cell can be grasped by calculating the degree of activity (ADMETOX) of the cell on the basis of the fluorescent image of the cell and judging whether this degree of activity is good or not. (2) The optical flow is calculated from the time differential image of the fluorescent image by an arithmetic operation, and the degree of activity of the cell is determined by making an inference for distinguishing the circulatory activity within the cell and the Brownian movement on the basis of this calculation, and it is judged whether the degree of activity is good or not, and the result of this judgment is obtained in the early stage of screening.

This embodiment will next be explained by using the constructional view shown in Fig. 4. The constructions and the operations of sensor section 100, control mechanical section 200, image processing section 300, plate 400, control section 500a and dispenser 800 are equal to those in the case of the above embodiment. Accordingly, their explanations are omitted here. The functions and the operations of judging section 700 and display section 600 will be explained next.

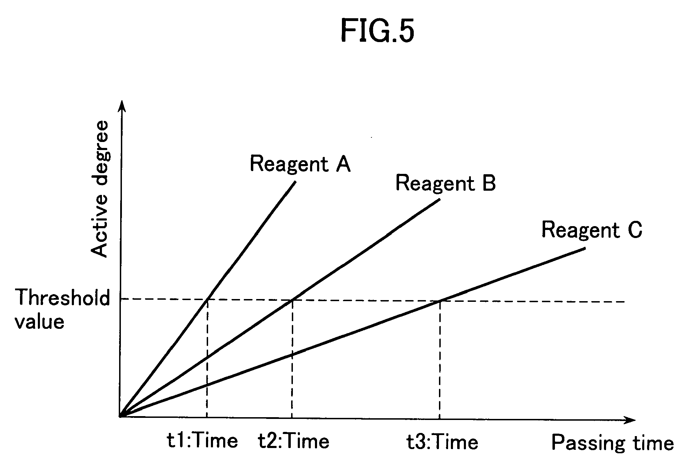

Similar to the above preferred embodiment, judging section 700 has a function for determining the degree of activity of the cell by using image data obtained in image processing section 300. Judging section 700 of this embodiment further judges whether the degree of activity calculated in this way is good or not. Fig. 5 schematically shows a time change of the degree of activity of the cell due to a difference in the kind of reagent. The axis of abscissa shows a passing time, and the axis of ordinate shows the degree of activity. In Fig. 5, line A shows the time change in the degree of activity of the cell of a well into which reagent A has been injected. Line B shows the time change in the degree of activity of the cell of a well into which reagent B has been injected. Line C shows the time change in the degree of activity of the cell of a well into which reagent C has been injected. Thus, the time change in the degree of activity is different depending on the kind of reagent. Such characteristics of degree of activity are also different depending on the density of the reagent.

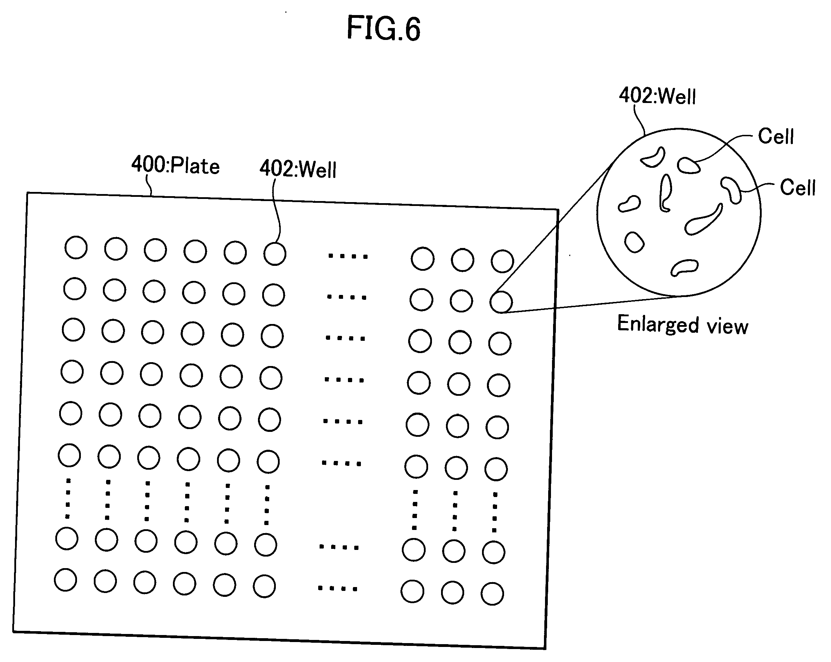

Judging section 700 applies a certain threshold value to such a degree of activity and judges whether the degree of activity of the cell is good or not, in other words, whether the effect of the reagent with respect to the cell is good or bad according to whether the degree of activity is the threshold value or more, or the threshold value or less. In this case, as illustrated by the enlarged view of Fig. 6, plural cells exist within one well 402. Judging section 700 sets the degree of activity of the well by for example, a maximum value or an average value of the degree of activity of each cell, and judges whether the degree of activity is good or not. The judging result as to whether the degree of activity is good or not, is outputted by adding the information of a display color corresponding to the goodness to the judging result so as to easily identify it. For example, the judging result is outputted by adding red information to the well having a degree of activity of the threshold value or more.

The judging result can be stored to a memory means, not shown in the figure, as necessary.

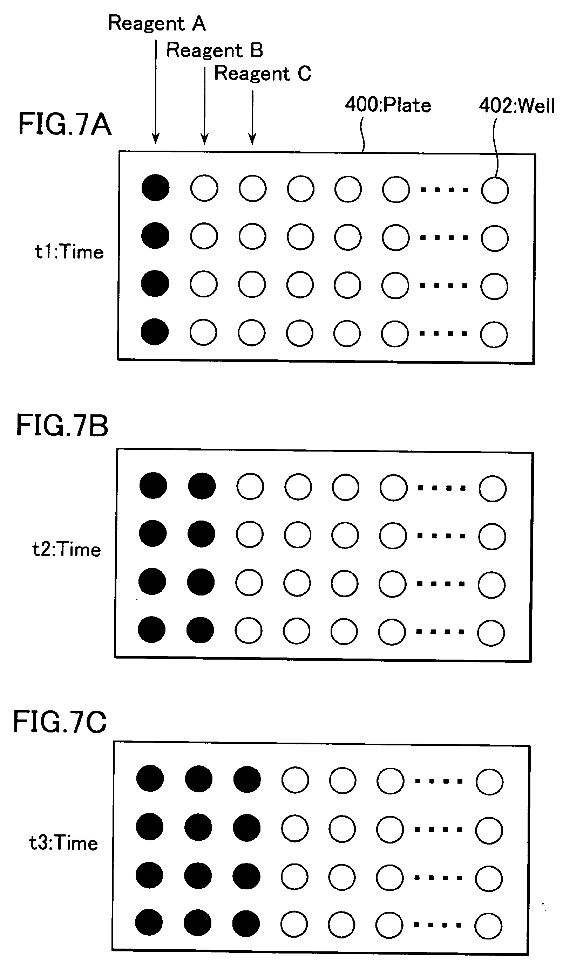

As shown in Fig. 7, display section 600 displays the judging result of the degree of activity with respect to each well 402 of plate 400. In this case, reagents A, B and C shown in Fig. 5, are injected into the first, second and third columns from the left. Figs. 7A, 7B and 7C show display examples of the respective judging results at times t1, t2, t3 of Fig. 3. In these figures, the wells of the threshold value or more in the degree of activity are shown by black circles.

Fig. 7 shows the display examples of the judging result when the density is different in accordance with each reagent. Fig. 7 shows a case in which the density sequentially becomes thin from the upper side to the lower side.

The degree of activity may also be judged to be good or not by using two threshold values or more.

The operation of the device in such a construction will be explained next. The operations until image data are obtained in image processing section 300, are equal to those in the above embodiment. Therefore, their explanations are omitted here. The image data processed by image processing section 300 are transmitted to judging section 700. Judging section 700 calculates the optical flow by the AI inferential engine from the differential image between the image of time (t) and the image of time (t+Δt) after a certain time has passed. Judging section 700 then calculates the degree of activity of the cell for each well.

Subsequently, judging section 700 judges whether the degree of activity of the cell is good or not for each well with a certain threshold value as a reference. In other words, the effect of the reagent with respect to the cell. With respect to the degree of activity of the threshold value or more, it is judged as good, and the judging result is outputted by adding, for example, red display color information.

The judging result is displayed in the format as shown in Fig. 7 in display section 600. In this figure, wells judged as good in the degree of activity are displayed in black. Figs. 7A, 7B and 7C display the respective judging results at times t1, t2, t3 corresponding to Fig. 5.

It is possible to easily judge and confirm whether the degree of activity of the cell is good or not, in other words, the effect of the reagent with respect to the cell by observing such displays.

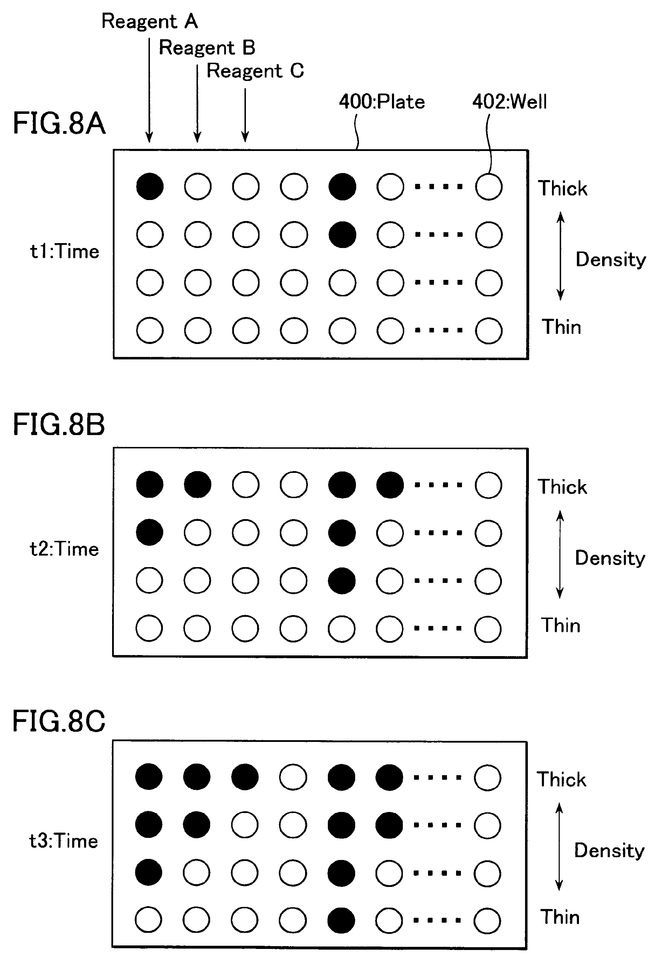

Fig. 8 shows a display pattern example of the judging result when the reagent of a different kind is simply injected to every column of the well. However, the display pattern becomes more complicated when a reagent of a different kind and density is injected into each cell.

Control section 500a can display information the injecting condition of the well, for example, the kinds, the densities, and so on, of the cell and the reagent injected into each well in display section 600 together with the display of the above judging result. Thus, whether the degree of activity of the cell is good or not for each well is automatically judged, and the judging result can be displayed in an easily recognizable display format.

The number of wells arranged in one plate is large and it is not easy to find the optimum well and the worst well from the plate after the measurement. There are also cases in which these wells are easily found in error. Therefore, in control section 500a, only a target well of plate 400 after the measurement is marked by operating dispenser 804 for marking and control mechanical section 200 on the basis of the judging result of judging section 700. For example, colored paint is attached to the vicinity of the target well on the plate surface.

More changes and modifications are included in the preferred embodiment shown in Fig. 4 and this embodiment. For example, the dispenser may be also operated at high speed by using the Piezo actuator instead of the electromagnetic pump.

Further, the number of plates 400 is not limited to one, but setting of the injecting condition, the observation and the judgment can be made continuously with respect to plural plates of conditions different from each other.

Further, image processing section 300 and judging section 700 are shown as independent constructional elements in the drawings, but may also be constructed so as to be included in control section 500a.

Further, in the embodiments, three-dimensional objective lens 130 is scanned in the Z-axis direction by Piezo actuator 140, but three-dimensional processing may also be performed by using multiheads with far, mid- and close focal positions, and so on.

Otherwise, the focal position of the objective lens with respect to the sample may also be varied by voltage control by inserting a varifocal lens of a voltage control type between objective lens 130 and confocal scanner 120 without operating objective lens 130 by the Piezo actuator.

Further, according to the invention, the excitation is performed by the laser beam. In an example which does not fall within the scope of the present invention, fluorescent light may be also generated from the sample by utilizing 2-photon absorption, irradiating infrared light as the excitation light and causing the 2-photon absorption. In accordance with this construction, it is sufficient to irradiate the infrared light having reduced light energy to the cell so that the influence of the light on the cell can be almost removed.

Further, the image of the sample may also be picked up by the camera by dyeing the cell using quantum dots, and exciting them by a short wavelength laser beam using a camera filter provided with spectroscopic characteristics having a discriminating function. Thus, serisitivity and discriminating ability can be easily improved.

Further, in an example which does not fall within the scope of the invention, the output image of the Nipkow system confocal scanner may also be detected by using a line sensor. In accordance with this construction, a coincident property and a higher resolution property can be simultaneously achieved. In this case, a multicolor line sensor may be also used as the line sensor.

Further, the living cell is observed intermittently over a long time by the confocal scanner, and the active state of the cell can be also trend-displayed.

An image (an image of long focal depth corresponding to a scanning thickness which is called a long focal image) obtained by exposing sectional image information of the sample (cell) obtained by scanning the objective lens in the optical axis direction by a required depth within the same frame period of the camera may be also used as the image utilized in image processing section 300.

In accordance with the preferred embodiments of the invention, the following effects are shown.
(1) The drug effect of each well can be rapidly and accurately grasped by judging the degree of activity of the cell. In this case, since each well is classified by color and is displayed on the display screen corresponding to the judging results of the degree of activity, it is possible to easily grasp whether the drug effect is good or not.
(2) In the judging, the degree of activity is judged by the inferential portion assembling a program for distinguishing the circulatory activity within the cell and the Brownian movement on the basis of the optical flow calculated from the time differential image. Therefore, the degree of activity of the cell can be judged without error.

Further, in accordance with this judgment of the degree of activity, the degree of activity can be rapidly judged in the early stage of screening, and screening can be performed easily at high speed.

## Claims

1. A screening device comprising:
a sensor section (100) including a laser light source (110), a Nipkow system confocal scanner (120), an objective lens (130) for converging light from said laser light source (110) onto a sample (401) of a plate (400) and a camera (150);
a control mechanical section (200),
an image processing section (300),
a central processing unit (500),
a display section (600),
a XYZ-drive controllable by said control mechanical section (200) for arranging a substrate stage, which is configured to place said plate (400) thereon, in a predetermined position with respect to said sensor section (100) and for moving the substrate stage suitably in the XYZ directions so as to observe each sample (401) provided on said plate (400);
wherein said Nipkow system confocal scanner (120) includes a microlens disk (122) for converging excitation light from said laser light source (110) to an individual light beams, a dichroic mirror (124) for transmitting the individual light beams and for reflecting fluorescent light generated from sample (401), and a pinhole disk (125) provided so as to pass the individual light beams through individual pinholes (126) to the objective lens (130);
a focal position variable means (140) for continuously or discontinuously moving a focal position of the objective lens (130) in the optical axis direction, that is the Z-axis direction;
said central processing section (500) being configured to control the screening device so that slice images of said sample in different sectional positions along the optical-axis direction are obtained at high speed in the Z-axis direction by the camera (150).

2. A screening device comprising:
a sensor section (100) including a laser light source (110), a Nipkow system confocal scanner (120), an objective lens (130) for converging light from said laser light source (110) onto a sample (401) of a plate (400) and a camera (150);
a control mechanical section (200),
an image processing section (300),
a central processing unit (500),
a display section (600),
a XYZ-drive controllable by said control mechanical section (200) for arranging a substrate stage, which is configured to place said plate (400) thereon, in a predetermined position with respect to said sensor section (100) and for moving the substrate stage suitably in the XYZ directions so as to observe each sample (401) provided on said plate (400);
wherein said Nipkow system confocal scanner (120) includes a microlens disk (122) for converging excitation light from said laser light source (110) to an individual light beams, a dichroic mirror (124) for transmitting the individual light beams and for reflecting fluorescent light generated from sample (401), and a pinhole disk (125) provided so as to pass the individual light beams through individual pinholes (126) to the objective lens (130);
a focal position variable means (140) for continuously or discontinuously moving a focal position of the objective lens (130) in the optical axis direction, that is the Z-axis direction;
said central processing section (500) being configured to control the screening device so that an image of a long focal depth corresponding to a scanning thickness is obtained by scanning the objective lens in the optical axis direction by a required depth within a frame period of the camera (150).

3. The screening device of claim 1 or 2, **characterized in that**
the focal position variable means (140) comprises a piezo actuator (140).

4. The screening device of claim 1 or 2, **characterized in that**
the focal position variable means (140) comprises a varifocal lens and means for controlling the voltage applied thereto between the objective lens (130) and said Nipkow system confocal scanner (120).

5. The screening device of claim 1 or 2, **characterized in that**
said Image processing section (300) is configured to receive an image signal from the camera (150), and to perform image processing and data processing for showing the degree of activity of a sample, including processing for making a statistical analysis of the fluorescent strength of the cell, kinetics, a histogram, or a correlation diagram.

6. The screening device of claim 5, **characterized in that** said Image processing section (300) is configured to form one two-dimensional image, that is an overlapping image, from the plural slice images obtained by the camera (150) and to classify the image by color in accordance with the difference in the expression amount of a fluorescent protein in the samples (401).

7. The screening device of claim 1 or 2, **characterized by** further comprising
at least one dispenser (800, 801, 802, 803) having a mechanism for respectively injecting a living cell fluorescent marked in advance and a reagent into each well of said plate (400).

## Patentansprüche

1. Screeningvorrichtung, aufweisend:
einen Sensorabschnitt (100) mit einer Laserlichtquelle (110), einem konfokalen Scanner eines Nipkow-Systems (120), einer Objektivlinse (130) zum Konvergieren von Licht von der Laserlichtquelle (110) auf eine Probe (401) einer Platte (400), und einer Kamera (150),
einen mechanischen Steuerungsabschnitt (200),
einen Bildverarbeitungsabschnitt (300),
eine zentrale Verarbeitungseinheit (500),
einen Anzeigeabschnitt (600),
einen XYZ-Antrieb, der durch den mechanischen Steuerungsabschnitt (200) steuerbar ist, zum Anordnen einer Substratstufe, welche so konfiguriert ist, dass die Platte (400) darauf angeordnet ist, an einer vorbestimmten Position mit Bezug auf den Sensorabschnitt (100), und zum geeigneten Bewegen der Substratstufe in den XYZ-Richtungen, damit jede auf der Platte (400) vorgesehene Probe (401) beobachtet wird,
wobei der konfokale Scanner eines Nipkow-Systems (120) eine Mikrolinsenscheibe (122) zum Konvergieren von Erregungslicht von der Laserlichtquelle (110) zu einzelnen Lichtstrahlen, einen dichromatischen Spiegel (124) zum Übertragen der einzelnen Lichtstrahlen und zum Reflektieren von fluoreszierendem Licht, das von der Probe (401) erzeugt ist, und eine Lochblendenscheibe (125), die so vorgesehen ist, dass die einzelnen Lichtstrahlen durch einzelne Lochblenden (126) zu der Objektivlinse (130) verlaufen, umfasst,
eine Fokusposition-Variationseinrichtung (140) zum kontinuierlichen oder diskontinuierlichen Bewegen einer Fokusposition der Objektivlinse (130) in der optischen Achsenrichtung, d.h. der Z-Achsenrichtung,
wobei der zentrale Verarbeitungsabschnitt (500) konfiguriert ist, um die Screeningvorrichtung so zu steuern, dass Schnittbilder der Probe an verschiedenen Schnittpositionen entlang der optischen Achsenrichtung in der Z-Achsenrichtung durch die Kamera (150) mit hoher Geschwindigkeit erhalten werden.

2. Screeningvorrichtung, aufweisend:
einen Sensorabschnitt (100) mit einer Laserlichtquelle (110), einem konfokalen Scanner eines Nipkow-Systems (120), einer Objektivlinse (130) zum Konvergieren von Licht von der Laserlichtquelle (110) auf eine Probe (401) einer Platte (400), und einer Kamera (150),
einen mechanischen Steuerungsabschnitt (200),
einen Bildverarbeitungsabschnitt (300),
eine zentrale Verarbeitungseinheit (500),
einen Anzeigeabschnitt (600),
einen XYZ-Antrieb, der durch den mechanischen Steuerungsabschnitt (200) steuerbar ist, zum Anordnen einer Substratstufe, welche so konfiguriert ist, dass die Platte (400) darauf angeordnet ist, an einer vorbestimmten Position mit Bezug auf den Sensorabschnitt (100), und zum geeigneten Bewegen der Substratstufe in den XYZ-Richtungen, damit jede auf der Platte (400) vorgesehene Probe (401) beobachtet wird,
wobei der konfokale Scanner eines Nipkow-Systems (120) eine Mikrolinsenscheibe (122) zum Konvergieren von Erregungslicht von der Laserlichtquelle (110) zu einzelnen Lichtstrahlen, einen dichromatischen Spiegel (124) zum Übertragen der einzelnen Lichtstrahlen und zum Reflektieren von fluoreszierendem Licht, das von der Probe (401) erzeugt ist, und eine Lochblendenscheibe (125), die so vorgesehen ist, dass die einzelnen Lichtstrahlen durch einzelne Lochblenden (126) zu der Objektivlinse (130) verlaufen, umfasst,
eine Fokusposition-Variationseinrichtung (140) zum kontinuierlichen oder diskontinuierlichen Bewegen einer Fokusposition der Objektivlinse (130) in der optischen Achsenrichtung, d.h. der Z-Achsenrichtung,
wobei der zentrale Verarbeitungsabschnitt (500) konfiguriert ist, um die Screeningvorrichtung so zu steuern, dass ein Bild einer langen Fokustiefe korrespondierend zu einer Scanning-Dicke erhalten wird, indem die Objektivlinse in der optischen Achsenrichtung um eine erforderliche Tiefe innerhalb einer Rahmendauer der Kamera (150) gescannt wird.

3. Screeningvorrichtung nach Anspruch 1 oder 2, **dadurch gekennzeichnet, dass**
die Fokusposition-Variationseinrichtung (140) einen Piezo-Aktuator (140) aufweist.

4. Screeningvorrichtung nach Anspruch 1 oder 2, **dadurch gekennzeichnet, dass**
die Fokusposition-Variationseinrichtung (140) eine Gleitsichtlinse und eine Einrichtung zum Steuern der zwischen der Objektivlinse (130) und dem konfokalen Scanner eines Nipkow-Systems (120) daran angelegten Spannung aufweist.

5. Screeningvorrichtung nach Anspruch 1 oder 2, **dadurch gekennzeichnet, dass**
der Bildverarbeitungsabschnitt (300) konfiguriert ist, um ein Bildsignal von der Kamera (150) zu empfangen und eine Bildverarbeitung und eine Datenverarbeitung zum Zeigen des Aktivitätsgrads einer Probe durchzuführen, einschließlich einer Verarbeitung zum Durchführen einer statistischen Analyse der fluoreszierenden Stärke der Zelle, Kinetik, eines Histogramms oder eines Korrelationsdiagramms.

6. Screeningvorrichtung nach Anspruch 5, **dadurch gekennzeichnet, dass** der Bildverarbeitungsabschnitt (300) konfiguriert ist, um ein zweidimensionales Bild, das ein überlappendes Bild ist, aus der Vielzahl von Schnittbildern, die durch die Kamera (150) erhalten werden, auszubilden, und das Bild durch Farbe gemäß der Differenz der Ausdrucksgröße eines fluoreszierenden Proteins in den Proben (401) zu klassifizieren.

7. Screeningvorrichtung nach Anspruch 1 oder 2, ferner **gekennzeichnet durch**
zumindest einen Dispenser (800, 801, 802, 803) mit einem Mechanismus zum jeweiligen Injizieren einer lebenden Zelle, die im Voraus fluoreszierend markiert ist, und eines Reagens in jede Senke der Platte (400).

## Revendications

1. Dispositif de criblage comprenant :
une section (100) formant sonde, comprenant une source (110) de lumière laser, un module (120) de balayage confocal de système de Nipkow, une lentille (130) formant objectif pour faire converger de la lumière de la source (110) de lumière laser sur un échantillon (401) d'une plaque (400) et une caméra (150) ;
une section (200) mécanique de commande,
une section (300) de traitement d'image,
une unité (500) centrale de traitement,
une section (600) d'affichage,
un entraînement XYZ pouvant être commandé par la section (200) mécanique de commande pour mettre une platine de substrat, qui est configurée pour y placer la plaque (400), dans une position déterminée à l'avance par rapport à la section (100) de sonde et pour déplacer la platine de substrat d'une manière appropriée dans les directions XYZ de façon à observer chaque échantillon (401) mis sur la plaque (400) ;
dans lequel le module (120) de balayage confocal du système de Nipkow comprend un disque (122) formant microlentille pour faire converger de la lumière d'excitation de la source (110) de lumière laser en un faisceau lumineux individuel, un miroir (124) dichroïque pour transmettre le faisceau lumineux individuel et pour réfléchir de la lumière fluorescente créée à partir de l'échantillon (401),
et un disque (125) à trous d'épingle prévu de manière à envoyer les faisceaux lumineux individuels à la lentille (130) formant objectif en les faisant passer dans des trous (126) d'aiguille individuels ;
un moyen (140) de variation de la position focale pour déplacer en continu ou en discontinu une position focale de la lentille (130) formant objectif dans la direction de l'axe, c'est-à-dire dans la direction de l'axe Z ;
la section (500) centrale de traitement étant configurée pour commander le dispositif de criblage de manière à ce que des images de tranches de l'échantillon en des positions en section différentes le long de la direction de l'axe optique soient obtenues à une vitesse grande dans la direction de l'axe Z par la caméra (150).

2. Dispositif de criblage comprenant :
une section (100) formant sonde, comprenant une source (110) de lumière laser, un module (120) de balayage confocal de système de Nipkow, une lentille (130) formant objectif pour faire converger de la lumière de la source (110) de lumière laser sur un échantillon (401) d'une plaque (400) et une caméra (150) ;
une section (200) mécanique de commande,
une section (300) de traitement d'image,
une unité (500) centrale de traitement,
une section (600) d'affichage,
un entraînement XYZ pouvant être commandé par la section (200) mécanique de commande pour mettre une platine de substrat, qui est configurée pour y placer la plaque (400), dans une position déterminée à l'avance par rapport à la section (100) de sonde et pour déplacer la platine de substrat d'une manière appropriée dans les directions XYZ de façon à observer chaque échantillon (401) mis sur la plaque (400) ;
dans lequel le module (120) de balayage confocal du système de Nipkow comprend un disque (122) formant microlentille pour faire converger de la lumière d'excitation de la source (110) de lumière laser en un faisceau lumineux individuel, un miroir (124) dichroïque pour transmettre le faisceau lumineux individuel et pour réfléchir de la lumière fluorescente créée à partir de l'échantillon (401),
et un disque (125) à trou d'épingle prévu de manière à envoyer les faisceaux lumineux individuels à la lentille (130) formant objectif en les faisant passer dans des trous (126) d'aiguille individuels ;
un moyen (140) de variation de la position focale pour déplacer en continu ou en discontinu une position focale de la lentille (130) formant objectif dans la direction de l'axe, c'est-à-dire dans la direction de l'axe Z ;
la section (500) centrale de traitement étant configurée pour commander le dispositif de criblage de manière à obtenir une image d'une longue profondeur focale correspondant à une épaisseur de balayage en balayant la lentille formant objectif dans la direction de l'axe optique d'une profondeur requise dans une période de trame de la caméra (150).

3. Dispositif de criblage suivant la revendication 1 ou 2, **caractérisé en ce que**
le moyen (140) de variation de la position focale comprend un actionneur (140) piézoélectrique.

4. Dispositif de criblage suivant la revendication 1 ou 2, **caractérisé en ce que**
le moyen (140) de variation de la position focale comprend une lentille à focale variable et des moyens de commande de la tension qui y est appliquée entre la lentille (130) formant objectif et le module (120) de balayage confocal du système de Nipkow.

5. Dispositif de criblage suivant la revendication 1 ou 2, **caractérisé en ce que**
la section (300) de traitement de l'image est configurée pour recevoir un signal d'image de la caméra (150) et pour effectuer un traitement d'image et un traitement de données pour montrer le degré d'activité d'un échantillon, y compris un traitement pour faire une analyse statistique de la force fluorescente de la cellule, la cinétique, un histogramme ou un diagramme de corrélation.

6. Dispositif de criblage suivant la revendication 5, **caractérisé en ce que**
la section (300) de traitement de l'image est configurée pour former une seule image en deux dimensions, qui est une image de chevauchement, à partir des plusieurs images de tranche obtenues par la caméra (150) et pour classer l'image par couleur en fonction de la différence dans la quantité d'expression d'une protéine fluorescente dans les échantillons (401).

7. Dispositif de criblage suivant la revendication 1 ou 2, **caractérisé en ce qu'**il comprend, en outre,
au moins un distributeur (800, 801, 802, 803) ayant un mécanisme pour injecter respectivement une cellule vivante marquée par fluorescence à l'avance et un réactif dans chaque puits de la plaque (400).
